# EUROPÄISCHE PATENTSCHRIFT

(11) **EP 2 982 327 B1**
(45) Veröffentlichungstag und Bekanntmachung des Hinweises auf die Patenterteilung: **24.11.2021**
(21) Anmeldenummer: 15180001.8
(22) Anmeldetag: 06.08.2015
(51) Int. Cl.: A61B 46/10, A61B 1/00

(54) **STERILHÜLLE FÜR EIN MEDIZINISCHES BEOBACHTUNGSINSTRUMENT SOWIE VERFAHREN ZUM BETREIBEN EINES MEDIZINISCHEN BEOBACHTUNGSINSTRUMENTS**
STERILE COVER FOR A MEDICAL OBSERVING INSTRUMENT AND METHOD FOR OPERATING A MEDICAL OBSERVATION INSTRUMENT
ENVELOPPE STERILE POUR UN INSTRUMENT D'OBSERVATION MEDICALE ET PROCEDE DE FONCTIONNEMENT D'UN INSTRUMENT D'OBSERVATION MEDICALE

(30) Priorität: 08.08.2014 DE 102014111354
(43) Veröffentlichungstag der Anmeldung: 10.02.2016
(73) Patentinhaber: Karl Storz SE & Co. KG, 78532 Tuttlingen (DE)
(72) Erfinder: Kupferschmid, Markus, 78532 Tuttlingen (DE); Czupalla, Christian, 78532 Tuttlingen (DE); Köhler, Benedikt, 78532 Tuttlingen (DE)
(74) Vertreter: Weidner Stern Jeschke

(56) Entgegenhaltungen:
- WO-A1-2014/025702
- US-A- 5 800 483
- US-A- 5 970 980
- US-A1- 2011 290 257

## Beschreibung

Die vorliegende Erfindung betrifft eine Sterilhülle für ein medizinisches Beobachtungsinstrument, insbesondere für ein Endoskop, ein Exoskop und/oder ein Operationsmikroskop, die zum Umhüllen mindestens einer wärmeabgebenden Komponente des medizinischen Beobachtungsinstruments ausgebildet ist, sowie ein Verfahren zum Betreiben eines medizinischen Beobachtungsinstruments.

Bei chirurgischen Eingriffen werden häufig medizinische Beobachtungsinstrumente verwendet, die ein Bild eines Operationsgebiets aufnehmen. Hierdurch kann beispielsweise eine vergrößerte Ansicht des Operationssitus oder, bei endoskopischen Operationen, eine intrakorporale Ansicht gewonnen werden. Ferner können hierdurch eine verbesserte und/oder ermüdungsfreie Wahrnehmung durch den Operateur und ggf. durch weitere Personen sowie die Aufzeichnung des Bilds beispielsweise für Dokumentationszwecke ermöglicht werden.

So ist es beispielsweise bekannt, mit einem Endoskop ein endoskopisches Bild eines körperinneren Hohlraums aufzunehmen, in dem ein chirurgischer Eingriff durchgeführt wird. Endoskope umfassen typischerweise einen starren oder flexiblen lang erstreckten Schaft, der zur Einführung in den Hohlraum geeignet ist, und in dessen distalem (d.h. beobachterfernen) Endbereich ein Objektiv zur Erzeugung eines Bildes eines Objektfelds in dem Hohlraum aufgenommen ist. Am proximalen (d.h. beobachternahen) Ende des Endoskops ist häufig eine elektronische Kamera zur Aufnahme des über einen Bildweiterleiter durch den Schaft zum proximalen Ende weitergeleiteten Bildes angeordnet. Da in der Regel in dem beobachteten Hohlraum nicht ausreichend Licht vorhanden ist, wird das notwendige Beleuchtungslicht üblicherweise im proximalen Endbereich des Endoskops erzeugt oder in dieses eingekoppelt und durch den Schaft zum distalen Ende weitergeleitet.

Weiterhin sind Operationsmikroskope bekannt, die die Erzeugung eines vergrößerten Bilds eines Operationssitus bei einer offenen chirurgischen Operation ermöglichen. Auch ein Operationsmikroskop verfügt häufig über eine elektronische Kamera sowie über eine Beleuchtungseinrichtung. Aus DE 10 2011 054 031 A1 ist eine Vorrichtung zum Beobachten und Beleuchten eines Objektfelds an einem Patienten von einer Stelle abseits des Körpers des Patienten bekannt, die am distalen Ende eines Schafts ein gegenüber dem Schaft erweitertes Kopfteil mit einer Beleuchtung zum Ausleuchten und einer Optik zum Beobachten des Objektfelds aufweist, wobei das aufgenommene Bild zu einem proximalen Ende des Schafts weitergeleitet wird, wo eine Videokamera angekoppelt sein kann. Eine derartige Vorrichtung, die die Beleuchtung und Beobachtung eines Operationsfelds bei einer chirurgischen Operation aus einem Arbeitsabstand von beispielsweise 25 bis 75 cm ermöglicht, wird als "Exoskop" bezeichnet. Exoskope der genannten Art werden von der Fa. KARL STORZ GmbH & Co. KG unter der Bezeichnung VITOM ® angeboten. Zum Halten eines Operationsmikroskops bzw. eines Exoskops in einer geeigneten Position zum Operationssitus kann eine Halterung vorgesehen sein, die einen je nach den gestellten Anforderungen verstellbaren und fixierbaren gelenkigen Arm aufweisen kann.

Die bei einer chirurgischen Operation im sterilen Bereich eingesetzten Instrumente müssen steril sein. Dies ist bei den zuvor erwähnten Beobachtungsinstrumenten, insbesondere wenn diese eine elektronische Kamera umfassen, nicht immer vollständig sicherzustellen, da diese häufig nicht für alle Sterilisationsverfahren, etwa für das Autoklavieren, geeignet sind. Daher ist es bekannt, derartige Beobachtungsinstrumente bzw. die nicht vollständig sterilen Teile der Beobachtungsinstrumente mit einer sterilen Hülle zu überziehen. Eine solche Sterilhülle oder "Drape" verhindert, dass Keime von dem nicht vollständig sterilen Teil des Beobachtungsinstruments in den Operationsbereich gelangen. Insbesondere verhindert die Sterilhülle, dass durch Berührung eines nicht vollständig sterilen Instruments eine Übertragung von Keimen auf mit dem Operationsfeld in Berührung kommende Oberflächen oder Materialien erfolgt.

Aus DE 202 21 380 U1 ist es bekannt, dass ein Operationsmikroskopsystem zur Verwendung in einer sterilen Arbeitsatmosphäre teilweise mit einem sterilen Abdecktuch bedeckt ist. Der durch das Abdecktuch eingeschlossene Raum ist mittels eines Bunds an einem Arm des Mikroskopständers abgedichtet. Dabei kann eine Saugeinheit, die eine Pumpe enthält, zum Entfernen von Medium aus dem Abdecktuch vorgesehen sein. Nachdem das sterile Abdecktuch um das Operationsmikroskop herum angeordnet und mittels des Bunds am Arm des Ständers befestigt worden ist, wird der von dem Abdecktuch eingeschlossene Raumbereich durch die Pumpe evakuiert. Um einen leichten und schnellen Wechsel des Tuchs zwischen zwei chirurgischen Operationen zu gestatten, kann das Abdecktuch durch Betreiben der Pumpe in einem umgesteuerten Zustand gelüftet werden, in dem Luft von außen in den von dem Abdecktuch umschlossenen Bereich gepumpt wird.

In WO 2014/025702 A1 ist ein chirurgisches Drape-System zum Bereitstellen eines sterilen Operationsfelds für einen Chirurgen bei ophthalmologischen Eingriffen offenbart. Dabei ist ein Drape über einer Stützstruktur angeordnet, um eine rechteckige Kammer zu schaffen, deren Boden durch ein chirurgisches Sieb gebildet werden kann. Ein Loch ist vorgesehen, um mit einem Mikroskop das unter dem Drape befindliche Operationsfeld zu beobachten. Mittels eines Ventilators kann gefilterte Luft in die Kammer eingeblasen werden, um kontaminierte Luft, die durch den Atem des Patienten eingebracht werden kann, vom Operationsfeld fort zu führen.

Aus US 5,970,980 ist ein steriler Videomonitor bekannt sowie eine Haltevorrichtung für den Videomonitor. Ein Ständer umfasst einen Monitorhalter, der eine Mehrzahl von Öffnungen aufweist, die mit einer Vakuumquelle kommunizieren, um ein steriles Drape eng auf dem Schirm des Videomonitors zu halten. Ein flexibler Körper des Drapes weist ein Lüftungsfilter auf mit einem Lüftungsfilterkörper, der dicht mit dem Drape verbunden ist, und einem Lüftungsfilterelement, wodurch das Einströmen von Luft ermöglicht wird, während Flüssigkeiten und unerwünschte Mikroorganismen zurückgehalten werden. In Zeiten, in denen keine Luft aus dem Innenraum des Drapes abgesaugt wird, wird hierdurch der Durchtritt von Verunreinigungen aus dem unsterilen Innenraum des Drapes in den sterilen Außenraum verhindert.

In US 5,800,483 ist ein steriles chirurgisch-thermisches Drape zum Erhalten eines sterilen Operationsgebiets mit einer thermischen Vorrichtung zum Regulieren der Körpertemperatur eines Patienten beschrieben. Dabei wird von einem Heiz- oder Kühlsystem ein heißes oder kaltes Gas rezirkulierend durch Kanäle der thermischen Vorrichtung geleitet.

Medizinische Beobachtungsinstrumente der beschriebenen Art, insbesondere Endoskope, Exoskope und Operationsmikroskope, weisen Komponenten auf, die sich beim Betrieb des Beobachtungsinstruments erwärmen und Wärme an ihre Umgebung abgeben. Derartige Komponenten sind beispielsweise elektronische Kameras und ggf. in das Beobachtungsinstrument integrierte Lichtquellen bzw. Lichtanschlüsse. Ist eine solche wärmeabgebende Komponente des Beobachtungsinstruments von einer Sterilhülle umgeben, so ist die Wärmeabgabe der Komponente in die Umgebung eingeschränkt oder nicht mehr möglich.

Hierdurch kann eine Überhitzung der betreffenden Komponente eintreten, die zu einer Verminderung der Bildqualität und/oder zu einer Beschädigung bzw. einer Verringerung der Lebensdauer der betreffenden Komponente führen kann.

Es ist Aufgabe der vorliegenden Erfindung, eine Sterilhülle für ein medizinisches Beobachtungsinstrument sowie ein Verfahren zum Betreiben eines medizinischen Beobachtungsinstruments anzugeben, bei dem die genannten Nachteile möglichst vermieden werden. Insbesondere ist es Aufgabe der vorliegenden Erfindung, eine Sterilhülle für ein medizinisches Beobachtungsinstrument und ein Verfahren zum Betreiben eines medizinischen Beobachtungsinstruments anzugeben, wobei eine Abführung der von einer wärmeabgebenden Komponente des Beobachtungsinstruments abgegebenen Wärme ermöglicht wird. Diese Aufgabe wird durch eine Vorrichtung gemäß Anspruch 1 sowie durch ein Verfahren gemäß Anspruch 14 gelöst.

Vorteilhafte Weiterbildungen der Erfindung ergeben sich aus den Unteransprüchen.

Eine erfindungsgemäße Sterilhülle ist zum zumindest teilweisen Umhüllen eines medizinischen Beobachtungsinstruments ausgebildet, insbesondere eines Endoskops, eines Exoskops oder eines Operationsmikroskops. Derartige medizinische Beobachtungsinstrumente sind oft nicht sterilisierbar oder umfassen einen oder mehrere Teile, die nicht oder nicht vollständig sterilisierbar sind. Die Verwendung von Sterilhüllen erlaubt zudem den schnell aufeinander folgenden Einsatz des unsterilen Medizinproduktes da auf den Sterilisationsschritt verzichtet werden kann. Die Sterilhülle ist zum Umhüllen des medizinischen Beobachtungsinstruments oder zumindest eines nicht oder nicht vollständig sterilisierbaren Teils desselben ausgebildet, um die Sterilität eines sterilen Bereichs, in dem oder in der Nähe dessen das medizinische Beobachtungsinstrument insbesondere bei einer chirurgischen Operation verwendet wird, zu gewährleisten.

Die erfindungsgemäße Sterilhülle ist vorzugsweise flexibel, insbesondere biegeschlaff, ausgebildet und besteht hierfür vorzugsweise aus einer flexiblen, transparenten Kunststofffolie. Eine derartige Sterilhülle ist insbesondere als Sterilüberzug ausgebildet, der auch als "Drape" bezeichnet wird. Die Sterilhülle kann aber auch bereichsweise flexibel und bereichsweise steif oder auch durchgehend steif ausgebildet sein, wobei die steifen Bereiche insbesondere aus einem transparenten Kunststoffmaterial bestehen. Die Sterilhülle kann Öffnungen beispielsweise zum Durchführen von Kabeln oder Haltearmen aufweisen. Die Betätigung von Bedienelementen des Beobachtungsinstruments, die ebenfalls von der Sterilhülle umhüllt werden, kann weiterhin möglich sein, beispielsweise durch die Kunststofffolie hindurch oder mit Hilfe besonderer Bedienbereiche der Sterilhülle. Hinsichtlich Größe und Form kann die Sterilhülle dem zu umhüllenden Beobachtungsinstrument angepasst sein, so dass die Sicht und die Bewegungsfreiheit eines Operateurs nicht mehr als notwendig eingeschränkt werden.

Die erfindungsgemäße Sterilhülle ist zum Umhüllen zumindest einer solchen Komponente des medizinischen Beobachtungsinstruments ausgebildet, die beim Betrieb Wärme abgibt. Eine solche wärmeabgebende Komponente kann beispielsweise eine elektronische Kamera bzw. eine Kameraeinheit oder auch eine Lichtquelle oder ein Lichtanschluss sein. Insbesondere ist die erfindungsgemäße Sterilhülle zum Umhüllen einer elektronischen Kamera bzw. einer Kameraeinheit ausgebildet. Eine solche Kamera bzw. Kameraeinheit, die weitere elektronische Bauelemente umfassen kann, ist in der Regel nicht sterilisierbar, insbesondere nicht autoklavierbar. Die Sterilhülle kann zum vollständigen oder teilweisen Umhüllen weiterer Komponenten des medizinischen Beobachtungsinstruments und/oder weiterer Vorrichtungen, etwa von Kabeln, Steuerungseinrichtungen oder Haltearmen des Beobachtungsinstruments, ausgebildet sein.

Erfindungsgemäß weist die Sterilhülle mindestens einen Lufteinlass, mindestens einen Luftauslass sowie Mittel zum Fördern und/oder Führen eines Luftstroms vom Lufteinlass durch die Sterilhülle zum Luftauslass auf. Die Sterilhülle ist somit insbesondere derart ausgebildet, dass durch den mindestens einen Lufteinlass zugeführte Luft zumindest durch einen Teilbereich eines von einer Außenwand der Sterilhülle umschlossenen Raumbereichs gefördert bzw. geführt werden kann und durch den mindestens einen Luftauslass wieder aus der Sterilhülle austreten kann. Bei einer einwandig ausgeführten Sterilhülle kann der Luftstrom zumindest durch einen Teilbereich eines von der Sterilhülle gebildeten Innenraums gefördert bzw. geführt werden. Bei einer mehrwandig ausgeführten Sterilhülle kann der Luftstrom durch einen von einer Innenwand der Sterilhülle gebildeten Innenraum und/oder durch einen zwischen der Innenwand und einer Außenwand gebildeten Zwischenraum gefördert bzw. geführt werden oder zumindest durch einen Teilbereich des Innen- bzw. Zwischenraums. Dabei ist der Innenraum zum Aufnehmen der mindestens einen wärmeabgebenden Komponente des medizinischen Beobachtungsinstruments ausgebildet, wobei diese vorzugsweise in einem von dem Luftstrom durchströmbaren Teilbereich des Innenraums aufgenommen werden kann. Abgesehen von dem mindestens einen Lufteinlass und dem mindestens einen Luftauslass ist die Sterilhülle vorzugsweise weitgehend gasdicht ausgebildet.

Als Mittel zum Fördern des Luftstroms können insbesondere Mittel zum Erzeugen eines Luftstroms vorgesehen sein, die eine aktive Luftzuführung und somit ein Einblasen von Luft in die Sterilhülle bewirken. Die Mittel zum Führen des Luftstroms umfassen insbesondere Mittel zum Leiten des Luftstroms in einer vorgegebenen Richtung oder entlang eines vorgegebenen Wegs innerhalb des Innenraums und/oder des Zwischenraums der Sterilhülle. Insbesondere kann durch die Mittel zum Fördern und/oder Führen des Luftstroms eine Luftströmung vom Lufteinlass entlang eines vorgegebenen Wegs innerhalb der Sterilhülle zum Luftauslass erzeugt bzw. ermöglicht werden. Der Luftstrom dient zur Abführung der von der mindestens einen wärmeabgebenden Komponente beim Betrieb abgegebenen Wärme in den Außenraum der Sterilhülle. Hierfür kann die Sterilhülle jeweils an die Bauart und/oder die Betriebsweise des medizinischen Beobachtungsinstruments angepasst sein. Insbesondere können die Mittel zum Fördern und/oder Führen des Luftstroms derart ausgebildet sein, dass ein ausreichend starker Luftstrom an einer wärmeabgebenden Oberfläche der Komponente oder nahe an einer wärmeabgebenden Oberfläche der Komponente entlanggeführt werden kann, um die abgegebene Wärme derart abzuführen, dass die Temperatur der Komponente im Betrieb im Wesentlichen konstant oder zumindest innerhalb eines zulässigen Temperaturbereichs bleibt.

Dadurch, dass die Sterilhülle einen Lufteinlass, einen Luftauslass und Mittel zum Fördern und/oder Führen eines Luftstroms durch die Sterilhülle aufweist, kann eine erzwungene Konvektion erzeugt bzw. ermöglicht werden, die die Abführung der Verlustwärme verbessert, die von einer von der Sterilhülle umschlossenen wärmeabgebenden Komponente des Beobachtungsinstruments abgegeben wird. Die Sterilhülle ist somit derart ausgebildet, dass ein Luftstrom durch die Sterilhülle zur Kühlung der mindestens einen wärmeabgebenden Komponente des Beobachtungsinstruments, die von der Sterilhülle umhüllt wird, erzeugt bzw. ermöglicht werden kann. Hierdurch kann eine unzulässige Erwärmung auch einer solchen wärmeabgebenden Komponente des medizinischen Beobachtungsinstruments vermieden werden, die nicht steril oder nicht sterilisierbar ist und die daher bei der Verwendung in oder nahe einem sterilen Bereich von einer Sterilhülle umhüllt sein muss.

Gemäß einer bevorzugten Ausführungsform der Erfindung umfassen die Mittel zum Fördern bzw. Führen des Luftstroms mindestens einen Ventilator und/oder mindestens eine Pumpe, wobei der Begriff "Ventilator" allgemein für Strömungsmaschinen und der Begriff "Pumpe" allgemein für Verdrängermaschinen steht, die den Luftstrom antreiben und somit insbesondere eine aktive Luftzuführung, d.h. ein Einblasen von Luft in die Sterilhülle, bewirken können. Vorzugsweise ist der Ventilator bzw. die Pumpe in die Sterilhülle integriert. Dadurch, dass die Sterilhülle einen Ventilator bzw. eine Pumpe zum Fördern und/oder Führen des Luftstroms umfasst, wird auf einfache Weise die Erzeugung einer erzwungenen Konvektion zur Wärmeabführung ermöglicht. Ferner werden die Anwendung der Sterilhülle und die Inbetriebnahme zusammen mit dem von dieser umhüllten medizinischen Beobachtungsinstrument erleichtert.

Zusätzlich oder alternativ kann die Sterilhülle einen Anschluss für eine externe aktive Luftzuführung aufweisen, wobei beispielsweise über einen anschließbaren Luftschlauch von einer externen Luftzuführungseinheit Luft zugeführt wird. Eine derartige externe Luftzuführungseinheit kann beispielsweise einer externen Lichtquelle zugeordnet sein, die in der Regel einen Ventilator zur Kühlung der Lichtquelle aufweist, der gemäß diesem Aspekt der Erfindung zur Erzeugung eines durch die Sterilhülle geführten Luftstroms zur Kühlung der wärmeabgebenden Komponente des medizinischen Beobachtungsinstruments genutzt werden kann. Auch hierdurch kann auf einfache Weise eine aktive Luftzuführung zur Erzeugung einer erzwungenen Konvektion zur Wärmeabführung ermöglicht werden.

Vorzugsweise umfasst die Sterilhülle weiterhin eine elektrische Energiequelle zur Versorgung des Ventilators bzw. der Pumpe, der bzw. die von der Sterilhülle umfasst ist. Die elektrische Energiequelle kann insbesondere eine Batterie oder ein Akku sein. Hierdurch wird eine autarke Energieversorgung insbesondere für eine aktive Luftzuführung der Sterilhülle ermöglicht, wodurch die Anwendung der Sterilhülle und die Inbetriebnahme zur Kühlung der mindestens einen wärmeabgebenden Komponente weiter erleichtert werden. Alternativ kann der Ventilator bzw. die Pumpe von einer externen Energiequelle versorgbar sein.

Vorzugsweise ist der Ventilator bzw. die Pumpe und/oder die Energiequelle zur Versorgung des Ventilators bzw. der Pumpe lösbar und somit wechselbar mit der Sterilhülle verbunden. Der Ventilator bzw. die Pumpe ist dabei insbesondere abgedichtet in eine hierfür ausgebildete Öffnung der Sterilhülle einsetzbar und aus der Öffnung wieder entnehmbar. Hierdurch wird eine Wiederverwendung des Ventilators bzw. der Pumpe und/oder der elektrischen Energiequelle ermöglicht, während die Sterilhülle im Übrigen als Einwegteil zur einmaligen Verwendung ausgebildet sein kann. Alternativ kann der Ventilator bzw. die Pumpe und/oder die elektrische Energiequelle ebenfalls als Einwegteil ausgebildet sein.

Gemäß einer bevorzugten Ausführungsform der Erfindung umfasst die Sterilhülle eine Mehrzahl von miteinander und mit dem Lufteinlass und dem Luftauslass in Strömungsverbindung stehenden Luftführungskammern, die zum Führen mindestens eines Teils des Luftstroms ausgebildet sind. Insbesondere ist ein von der Sterilhülle umschlossener Innenraum und/oder ein zwischen einer Innenwand und einer Außenwand der Sterilhülle gebildeter Zwischenraum in eine Mehrzahl von derartigen Luftführungskammern geteilt. Hierdurch wird eine Führung mindestens eines Teils des Luftstroms entlang eines vorbestimmten Wegs durch die Sterilhülle ermöglicht. Insbesondere kann die Mehrzahl von Luftführungskammern derart ausgebildet sein, dass eine Trennung der einströmenden und der ausströmenden Luft in der Art erfolgt, dass durch den Lufteinlass einströmende Luft zu einer wärmeabgebenden Oberfläche der wärmeabgebenden Komponente geführt wird, dort Wärme aufnimmt und danach zum Luftauslass geführt wird, ohne sich mit der einströmenden Luft zu vermischen. Auf diese Weise ist eine besonders effiziente Abführung der Verlustwärme der wärmeabgebenden Komponente des medizinischen Beobachtungsinstruments erreichbar.

Vorzugsweise ist die Sterilhülle zumindest bereichsweise mehrwandig, insbesondere doppelwandig ausgebildet, wobei zwischen einer Innenwand und einer Außenwand mindestens eine Luftführungskammer zum Führen mindestens eines Teils des Luftstroms durch den zwischen der Innen- und der Außenwand gebildeten Zwischenraum angeordnet ist. Die Luftführungskammer kann durch die Innenwand und die Außenwand und/oder eine oder mehrere zwischen der Innen- und der Außenwand angeordnete Zwischenwände begrenzt werden. Eine derartige Luftführungskammer kann insbesondere als Luftkanal ausgebildet sein, durch den mindestens ein Teil des Luftstroms vom mindestens einen Lufteinlass zum mindestens einen Luftauslass geführt wird. Zusätzlich kann ein Teil des Luftstroms durch den von der Innenwand der Sterilhülle umschlossenen Innenraum geführt werden; alternativ kann der Innenraum im Wesentlichen gasdicht abgeschlossen sein, so dass ein Wärmeübergang von der wärmeabgebenden Komponente zur Innenwand der Sterilhülle durch thermische Konvektion und von dort die Wärmeabführung durch den Luftstrom und somit durch erzwungene Konvektion erfolgt. Dadurch, dass zwischen der Innenwand und der Außenwand mindestens eine Luftführungskammer zum Führen mindestens eines Teils des Luftstroms durch den Zwischenraum zwischen der Innen- und der Außenwand vorgesehen ist, wird eine besonders effiziente Wärmeabführung ermöglicht. Sofern ein von der Innenwand umschlossener Bereich des Innenraums, in dem die mindestens eine wärmeerzeugende Komponente angeordnet werden kann, im Wesentlichen gasdicht ausgebildet ist, sind besonders hohe Sterilitätsanforderungen erfüllbar bzw. können niedrigere Anforderungen an die Sterilität der darin aufgenommenen Komponente des Beobachtungsinstruments gestellt werden; sofern andererseits ein Teil des Luftstroms durch den betreffenden Bereich des Innenraums geführt wird, ist eine weiter verbesserte Wärmeabführung erzielbar.

Weiterhin ist es bevorzugt, dass der Luftstrom in der mindestens einen Luftführungskammer turbulent geführt ist. Hierfür können beispielsweise Hindernisse, Vorsprünge, Verengungen, Krümmungen und/oder Aufrauhungen innerhalb der Luftführungskammer bzw. eines Luftkanals vorgesehen sein, wodurch die Turbulenz der Luftströmung vergrößert wird. Die turbulente Luftströmung ermöglicht eine besonders effiziente Wärmeaufnahme durch den Luftstrom und damit eine besonders effiziente Abführung der von der wärmeabgebenden Komponente erzeugten Verlustwärme.

Vorzugsweise ist die Sterilhülle derart ausgebildet, dass in der Sterilhülle ein Überdruck erzeugbar ist, beispielsweise durch eine aktive Luftzuführung mittels eines Ventilator oder einer Pumpe. Vorzugsweise kann bei einer zumindest bereichsweise flexibel bzw. biegeschlaff ausgebildeten Sterilhülle durch den erzeugten Überdruck innerhalb der mindestens einen Luftführungskammer bzw. im Innenraum eine Stabilisierung der Form der Sterilhülle erreichbar sein, die die Handhabung der Sterilhülle mit dem von dieser zumindest teilweise umschlossenen Beobachtungsinstrument erleichtert. Insbesondere ist in dem von einer Innenwand der Sterilhülle umschlossenen Innenraum bzw. in mindestens einer Luftführungskammer im Innenraum und/oder im Zwischenraum zwischen der Innen- und einer Außenwand ein hierfür ausreichender Überdruck erzeugbar. So kann beispielsweise ein Strömungsquerschnitt der mindestens einen Luftführungskammer und/oder der mindestens eine Luftauslass derart ausgebildet sein, dass bei einer aktiven Luftzuführung aufgrund des Strömungswiderstands ein entsprechender Überdruck in der mindestens einen Luftführungskammer bzw. im Innenraum entsteht. Die Sterilhülle ist dabei insbesondere derart ausgebildet, dass sie dann, wenn die mindestens eine Luftführungskammer durch den Überdruck "aufgeblasen" ist, eine gewünschte Form annimmt, die die Handhabung erleichtert und die Sicht und die Bewegungsfreiheit des Operateurs nicht behindert. Die Sterilhülle ist in vorteilhafter Weise ferner derart ausgebildet, dass bei Erzeugung eines für die Formstabilisierung ausreichenden Überdrucks gleichzeitig ein Luftstrom mit einer ausreichenden Wärmeabführungswirkung erzeugt wird. Hierdurch wird die Anwendbarkeit der Sterilhülle weiter erleichtert.

Gemäß einer bevorzugten Ausführungsform der Erfindung ist die Sterilhülle zumindest bereichsweise flexibel und mehrwandig, zumindest doppelwandig mit einer Innen- und einer Außenwand ausgebildet, wobei zwischen der Innen- und der Außenwand mindestens eine Stützkammer angeordnet ist, innerhalb derer durch eine aktive Luftzuführung ein Überdruck erzeugt werden kann. Die Stützkammer kann durch die Innenwand und die Außenwand und/oder eine oder mehrere zwischen der Innen- und der Außenwand angeordnete Zwischenwände begrenzt werden. Die mindestens eine Stützkammer ist nicht von einem Luftstrom durchströmt, ist aber derart ausgebildet, dass durch den Überdruck die Kammer in einer Form "aufgeblasen" und dadurch stabilisiert wird, die der zuvor biegeschlaffen Sterilhülle insgesamt eine gewünschte Form gibt. Die mindestens eine Stützkammer stellt daher im unter Überdruck stehenden Zustand eine Stützstruktur dar, die die Sterilhülle insgesamt stabilisiert. In besonders bevorzugter Weise ist eine Mehrzahl von Stützkammern vorgesehen, die eine Säulen- oder eine Wabenstruktur bilden, wodurch eine besonders wirksame Stabilisierung der Sterilhülle ermöglicht wird. Die hierdurch stabilisierte Form ist insbesondere an die Form und die Größe des von der Sterilhülle zumindest teilweise umschlossenen medizinischen Beobachtungsinstruments angepasst. Die Form ist vorzugsweise derart bestimmt, dass eine besonders wirksame Durchströmung der mindestens einen Luftführungskammer gewährleistet wird, wobei durch die stabilisierte Formgebung ggf. eine turbulente Strömung der Luft in der mindestens einen Luftführungskammer erzwungen werden kann. Hierdurch ist auf einfache Weise eine weiter verbesserte Wärmeabführungswirkung sowie eine verbesserte Handhabung der Sterilhülle erzielbar, wobei im unbenutzten Zustand die Sterilhülle eine beliebige Form annehmen kann, so dass die Lagerung vereinfacht wird.

Gemäß einer weiteren bevorzugten Ausführungsform weist die Sterilhülle eine selbststabilisierende Stützstruktur auf, die beispielsweise durch Stangen oder Drähte gebildet wird, die in die Sterilhülle eingelagert sind. Auch hierdurch können eine Stabilisierung der Sterilhülle und eine Verbesserung der Luftströmung in der mindestens einen Luftführungskammer erreichbar sein, wobei die Sterilhülle insbesondere an die Form und Größe des von der Sterilhülle zumindest teilweise umschlossenen Beobachtungsinstruments angepasst ist.

Alternativ oder zusätzlich kann die Sterilhülle bereichsweise oder auch durchgehend steif, insbesondere eigensteif und somit selbststabilisierend, ausgebildet sein und hierfür zumindest bereichsweise aus einem festen, vorzugsweise transparenten Kunststoffmaterial bestehen. Das Kunststoffmaterial ist vorzugsweise ausreichend dickwandig ausgeführt, so dass die Sterilhülle ohne weitgehende Verformung gegriffen und gehandhabt werden kann. Die Sterilhülle kann aus mindestens zwei Teilen bestehen, etwa aus zwei Hälften, die zum Aufnehmen und zum Entnehmen der zu umhüllenden Komponente des medizinischen Beobachtungsinstrumentes voneinander getrennt werden können und zum Umhüllen der Komponente im Wesentlichen luftdicht miteinander verbunden werden können, beispielsweise durch Zusammenstecken. Sofern die umhüllte Komponente des Beobachtungsinstruments Bedienelemente aufweist, können diese mit Hilfe von entsprechenden Bedienbereichen der Sterilhülle betätigbar sein, wobei die Bedienbereiche beispielsweise flexible Bereiche einer bereichsweise steifen Sterilhülle sein können. Eine zumindest bereichsweise steif ausgebildete Sterilhülle kann für die Handhabbarkeit des Beobachtungsinstruments in oder nahe einem sterilen Bereich vorteilhaft sein.

Erfindungsgemäß ist der Luftauslass mit einem Filter sowie in bevorzugter Weise mit einem Ventil versehen. Das Filter ist vorzugsweise derart ausgebildet, das es undurchlässig für Schmutzpartikel und Keime ist, die sich von einer Oberfläche eines von der Sterilhülle umhüllten unsterilen Instruments lösen könnten. Weiterhin ist der Luftaustritt insbesondere derart angeordnet, dass die austretende Luft aus dem Bereich des mit dem endoskopischen Beobachtungs- instrument beobachtbaren Objektfelds, insbesondere eines Operationssitus, entfernt wird, d.h. von diesem fort gerichtet aus dem Luftaustritt ausströmt und/oder der Luftaustritt an einem beim Betrieb des Beobachtungsinstruments vom Objektfeld bzw. vom Operationssi- tus fort gerichteten oder entfernten Ende der Sterilhülle angeordnet ist. Hierdurch kann ein Eindringen von Schmutz oder Keimen in den Bereich des Operationssitus noch sicherer vermieden werden.

Das Filter bzw. das Ventil ist vorzugsweise ferner derart ausgebildet, dass bei einer aktiven Luftzuführung innerhalb der mindestens einen Luftführungskammer und/oder der mindestens einen Stützkammer ein Überdruck erzeugbar ist, um wie oben beschrieben bei einer zumindest bereichsweise flexiblen Sterilhülle eine Stützwirkung zu erzielen und die Sterilhülle in einer gewünschten Form zu stabilisieren. Hierfür kann das Ventil auch beispielsweise einstellbar ausgebildet sein.

Gemäß einer weiteren bevorzugten Ausführungsform ist die Sterilhülle zumindest teilweise aus einem luftdurchlässigen Material ausgebildet, insbesondere ist eine Außenwand der Sterilhülle zumindest bereichsweise aus dem luftdurchlässigen Material ausgebildet, das mindestens einen Luftaustritt bildet. Ein derartiges Material kann beispielsweise eine Folie mit einer Mikroporosität sein, die den Austritt von Luft ermöglicht, jedoch den Durchtritt von Schmutz und/oder Keimen verhindert. Hierdurch wird es ermöglicht, den Luftstrom durch die Sterilhülle in besonders einfacher Weise zu führen und insbesondere einen Luftaustritt aus der Sterilhülle flächig auszubilden und in beliebigen Bereichen der Sterilhülle anzuordnen. Hierdurch kann eine Überwärmung einzelner Bereiche der Sterilhülle besonders sicher verhindert werden. Eine Kontamination des Operationssitus kann dabei weitestgehend vermieden werden, da selbst bei einem unsterilen Beobachtungsinstrument, das von der Sterilhülle umschlossen ist, Keime nur in geringem Maße in den Luftstrom gelangen und diese zusätzlich beim Durchtritt durch das poröse Material der Sterilhülle zurückgehalten werden. Trotz der Luftdurchlässigkeit des Materials kann daher eine Verschmutzung des Beobachtungsinstruments bei der Operation ebenfalls vermieden werden.

Erfindungsgemäß umfasst die Sterilhülle ein Deckglas, das an einem distalen Fenster des von der Sterilhülle umschlossenen Beobachtungsinstruments fixierbar ist. Das distale Fenster ist gasdicht in die Sterilhülle eingesetzt. Hierdurch wird einerseits eine ungestörte Beobachtung mit dem Beobachtungsinstrument und andererseits eine Fixierung der Sterilhülle am Beobachtungsinstrument ermöglicht. Ferner kann eine Verschmutzung des Beobachtungsinstruments bei einer Operation vermieden werden.

Gemäß einer alternativen, nicht beanspruchten Ausführungsform weist die Sterilhülle eine Öffnung auf, die an ein distales Fenster des Beobachtungsinstruments gasdicht anschließbar ist. Hierfür kann die Öffnung insbesondere von einer Dichtung umgeben sein, die zum Anschluss an das distale Fenster des Beobachtungsinstruments, das durch ein dem Beobachtungsinstrument zugeordnetes Deckglas abgeschlossen sein kann, angepasst ist. Hierdurch wird ebenfalls einerseits eine ungehinderte Sicht mit dem Beobachtungsinstrument auf den Operationssitus ermöglicht und andererseits eine Befestigung der Sterilhülle an dem von dieser zumindest teilweise umhüllten medizinischen Beobachtungsinstrument ermöglicht.

Als alternative Befestigungsmöglichkeit können eine oder mehrere Klebeflächen auf einer Innenseite der Sterilhülle angeordnet sein. Auch hiermit kann auf einfache Weise eine Fixierung der Sterilhülle an dem von dieser umhüllten medizinischen Beobachtungsinstrument ermöglicht werden, um die Handhabung des Beobachtungsinstruments zu erleichtern. Gemäß einem erfindungsgemäßen Verfahren zum Betreiben eines medizinischen Beobachtungsinstruments, insbesondere eines Exoskops oder Operationsmikroskops, wird das medizinische Beobachtungsinstrument oder zumindest eine wärmeabgebende Komponente des Beobachtungsinstruments von einer Sterilhülle umhüllt, die wie oben beschrieben ausgebildet ist; ggf. kann die Sterilhülle zuvor durch Einsetzen eines Ventilators oder einer Pumpe zusammen mit einer elektrischen Energiequelle vorbereitet worden sein. Weiter wird das nunmehr zumindest teilweise in der Sterilhülle aufgenommene medizinische Beobachtungsinstrument zur Beobachtung eines Operationssitus angeordnet, beispielsweise wird ein Exoskop oder ein Operationsmikroskop an einem Haltearm befestigt, der teilweise ebenfalls von der Sterilhülle umhüllt werden kann, und in geeignetem Abstand über dem Operationssitus gehalten. Bei einem Endoskop kann beispielsweise eine Videokamera, die mit der Sterilhülle umhüllt worden ist, an das Endoskop angeschlossen werden. Sodann wird die wärmeabgebende Komponente, etwa eine Videokamera, in Betrieb genommen, wobei Verlustwärme freigesetzt wird. Zur Abführung der Verlustwärme wird eine aktive Luftzuführung der Sterilhülle in Betrieb gesetzt, etwa ein Ventilator oder eine Pumpe, die in die Sterilhülle integriert sind, oder es wird eine externe Luftzuführungseinrichtung über einen Luftschlauch an den Lufteinlass der Sterilhülle angeschlossen und in Betrieb genommen. Dadurch wird ein Luftstrom vom Lufteinlass durch die Sterilhülle geleitet, insbesondere durch den von einer Innenwand der Sterilhülle umschlossenen Innenraum und/oder durch einen zwischen der Innenwand und einer Außenwand der Sterilhülle gebildeten Zwischenraum, wobei der Luftstrom vorzugsweise durch Luftführungskammern an einer wärmeabgebenden Oberfläche der wärmeabgebenden Komponente entlang oder in einer Umgebung der Komponente geführt wird. Hierdurch kann eine verbesserte Abführung der beim Betrieb der wärmeabgebenden Komponente freigesetzten Verlustwärme ermöglicht und eine Überhitzung der wärmeabgebenden Komponente beim Betrieb vermieden werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1 ein Ausführungsbeispiel einer erfindungsgemäßen Sterilhülle mit einem von dieser umhüllten Exoskop in schematischer Schnittdarstellung;
Fig. 2 ein nicht beanspruchtes Ausführungsbeispiel einer Sterilhülle mit einer von dieser umhüllten Videokamera eines Endoskops in schematischer Schnittdarstellung.

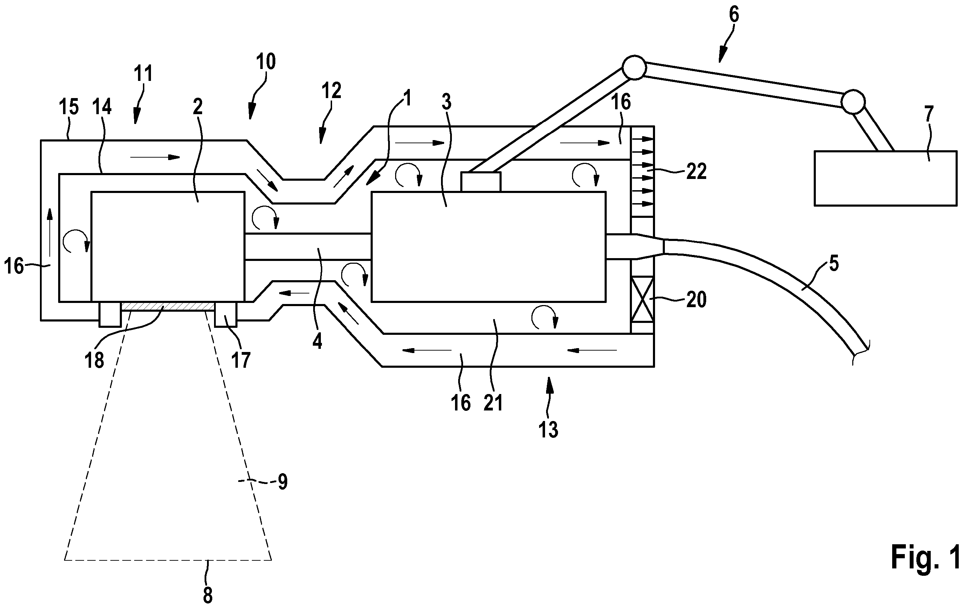

Wie in Fig. 1 in einem schematischen Längsschnitt dargestellt, ist ein Exoskop 1 beim Einsatz im Operationssaal von einer Sterilhülle 10 umhüllt. Die Sterilhülle 10 kann flexibel, insbesondere biegeschlaff, oder steif, insbesondere eigensteif, ausgebildet sein. Das Exoskop 1 umfasst einen Kopf 2, in dem eine Beleuchtungseinrichtung zur Beleuchtung eines Objektfelds 8, etwa eines Operationssitus, und ein Objektiv angeordnet sind. Das Exoskop 1 umfasst weiterhin eine Kameraeinheit 3, die über einen Schaft 4 mit dem Kopf 2 des Exoskops 1 verbunden ist. In dem Schaft 4 verlaufen elektrische Leitungen zur Versorgung der in dem Kopf 2 angeordneten Beleuchtungseinrichtung sowie ein Bildweiterleiter, der beispielsweise durch ein geordnetes Faserbündel oder durch Relaislinsensysteme gebildet werden kann, zur Weiterleitung des vom Objektiv aufgenommenen Bilds des Objektfelds 8. Die Kameraeinheit 3 ist über ein Versorgungskabel 5 mit einer nicht dargestellten Versorgungs- und Auswerteeinrichtung verbunden. Das Exoskop 1 wird von einem als Gelenkarm ausgebildeten Haltearm 6, der an einer festen Struktur 7 des Operationssaals ansetzt, in einer für die Beobachtung des Objektfelds 8 geeigneten Position gehalten.

Da das Exoskop 1 nur eingeschränkt sterilisierbar ist, ist es von einer Sterilhülle 10 umhüllt. Diese weist einen distalen Abschnitt 11 auf, der zur Umhüllung des Kopfs 2 des Exoskops 1 ausgebildet ist, einen Schaftabschnitt 12, der den Schaft 4 einschließt, und einen proximalen Abschnitt 13, dessen Form und Größe zur Umhüllung der Kameraeinheit 3 des Exoskops 1 angepasst sind. Die Sterilhülle 10 ist doppelwandig ausgebildet, wobei zwischen der Innenwand 14 und der Außenwand 15 von Luft durchströmbare bzw. mit Luft füllbare Kammern angeordnet sind. In Fig. 1 ist zwischen der Innenwand 14 und der Außenwand 15 eine von Luft durchströmbare Kammer, die als Luftkanal 16 ausgebildet ist, gezeigt. Der Luftkanal 16 ist durch in Fig. 1 nicht dargestellte Zwischenwände gegen weitere Luftführungskanäle sowie ggf. gegen Stützkammern, die ebenfalls zwischen der Innenwand 14 und der Außenwand 15 angeordnet sein können, abgetrennt.

Der distale Abschnitt 11 der Sterilhülle 10 weist ein Deckglas 18 auf, das gasdicht in einen Kunststoffeinsatz 17 eingefügt ist, der wiederum gasdicht mit der Innenwand 14 und der Außenwand 15 verbunden ist. Der Kunststoffeinsatz 17 und das Deckglas 18 sind derart bemessen und angeordnet, dass die im Kopf 2 des Exoskops 1 aufgenommene Beleuchtungseinrichtung sowie das Objektiv durch das Deckglas 18 hindurch wirken können, wie in Fig. 1 symbolisch durch den Lichtkegel 9 angedeutet ist. Der Luftkanal 16 ist um das Deckglas 18 bzw. um den Kunststoffeinsatz 17 herum geführt.

Der proximale Abschnitt 13 der Sterilhülle 10 weist eine in Fig. 1 nicht näher dargestellte Öffnung zur Durchführung des Haltearms 6 sowie eine ebenfalls nicht näher dargestellte Öffnung zur Durchführung des Versorgungskabels 5 auf. Bei beiden Öffnungen sind in Fig. 1 nicht dargestellte Dichtmittel vorgesehen, beispielsweise Gummizüge, so dass die Sterilhülle 10 gasdicht an dem Haltearm 6 bzw. dem Versorgungskabel 5 abschließt. Weiterhin umfasst die Sterilhülle 10 in ihrem proximalen Abschnitt 13 einen Ventilator 20, der zur Zuführung von Luft in den Luftkanal 16 sowie gegebenenfalls in den Innenraum 21, der von der Innenwand 14 der Sterilhülle 10 umschlossen wird und in dem das Exoskop 1 aufgenommen ist, betrieben werden kann. Sofern die Sterilhülle 10 flexibel ausgebildet ist, kann der Ventilator 20 zum Zuführen von Luft in die zwischen der Innenwand 14 und der Außenwand 15 angeordneten, in Fig. 1 nicht gezeigten Stützkammern und zur Erzeugung eines Überdrucks in diesen Kammern ausgebildet sein, so dass hierdurch die Sterilhülle 10 in einer das Exoskop 1 in einem Abstand umschließenden Form stabilisiert wird. Sofern die Sterilhülle 10 eigensteif ausgebildet ist, umschließt sie das Exoskop 1 ebenfalls in einem Abstand, wobei das Exoskop 1 in der Sterilhülle 10 gehalten wird und mit Hilfe der grifffesten Sterilhülle 10 gehandhabt werden kann. Der Ventilator 20 umfasst eine integrierte Batterie, die diesen mit elektrischer Energie versorgt, und ist lösbar, jedoch abgedichtet, in eine entsprechende Öffnung der Sterilhülle 10 eingesetzt.

Weiterhin umfasst die Sterilhülle 10 in ihrem proximalen Abschnitt 13 eine Austrittsöffnung, in der ein Filter 22 angeordnet ist, durch das die in den Luftkanal 16 sowie ggf. in den Innenraum 21 zugeführte Luft nach außen entweichen kann. Insbesondere in dem Fall, dass Luft aus dem Innenraum 21 durch die Austrittsöffnung bzw. das Filter 22 austritt, ist dieses derart ausgebildet, dass Schmutzpartikel und Keime, die sich von dem nicht vollständig sterilisierten Exoskop 1 lösen könnten, zurückgehalten werden. Wie in Fig. 1 angedeutet, ist die Austrittsöffnung mit dem Filter 22 am proximalen Ende des proximalen Abschnitts 13 der Sterilhülle 1 angeordnet, d.h. an dem vom Kopf 2 des Exoskops 1 entfernten Ende, so dass die in den Außenraum austretende Luft vom Objektfeld 8 fort geführt wird.

Zur Vorbereitung der Verwendung des Exoskops 1 wird dieses mit der Sterilhülle 10 überzogen bzw. in diese eingesetzt. Hierfür weist die Sterilhülle 10 eine Öffnung auf, die beispielsweise die Öffnung sein kann, durch die der Haltearm 6 geführt ist und die so bemessen ist, dass das Exoskop 1 in den Innenraum 21 der Sterilhülle 10 eingeführt werden kann. In diesem Zustand ist der Ventilator 20 noch nicht in Betrieb, so dass weder der Innenraum 21 noch der Luftkanal 16 oder ggf. die Stützkammern, die zwischen der Innenwand 14 und der Außenwand 15 der Sterilhülle 10 abgeteilt sind, mit einem Überdruck beaufschlagt sind. Wenn die Sterilhülle 10 im Wesentlichen aus einer flexiblen, durchsichtigen Kunststofffolie besteht, ist daher biegeschlaff und leicht handhabbar. Sofern die Sterilhülle 10 steif ausgebildet ist und hierfür aus einem festen Kunststoffmaterial besteht, kann diese aus zwei Hälften bestehen, die nach Einsetzen des Exoskops 1 luftdicht miteinander verbunden werden können (nicht in Fig. 1 dargestellt). Das in die Sterilhülle 10 integrierte Deckglas 18 wird vor der Beleuchtungsoptik und dem Objektiv des Exoskops 1 angeordnet und der Kunststoffeinsatz am Kopf 2 des Exoskops 1 fixiert, wofür der Kunststoffeinsatz 17 und der Kopf 2 miteinander zusammenwirkende Arretierungselemente aufweisen können. Ist das Exoskop 1 in den Innenraum 21 der Sterilhülle 10 eingeführt worden, so wird das Verbindungskabel 5 an das Exoskop 1 angeschlossen und die Öffnungen der Sterilhülle 10, durch die das Verbindungskabel 5 und der Haltearm 6 geführt sind, weitgehend gasdicht abgedichtet. Sofern der Ventilator 20 und das Filter 22 nicht fest in die Sterilhülle 10 integriert sind, werden diese vor Inbetriebnahme in diese eingesetzt.

Wenn das Exoskop 1 in Betrieb genommen wird, so dass im Bereich des Kopfs 2 und der Kameraeinheit 3 Wärme freigesetzt wird, wird auch der Ventilator 20 in Betrieb genommen. Der Ventilator 20 fördert Luft in den Luftkanal 16 und ggf. in den von der Innenwand 14 umschlossenen Innenraum 21. Sofern die Sterilhülle flexibel ausgebildet ist, kann der Ventilator 20 auch in die zwischen der Innenwand 14 und der Außenwand 15 gebildeten Stützkammern Luft fördern; der in den Stützkammern erzeugte Überdruck stabilisiert die Sterilhülle 10 in der vorgegebenen Form, die zum Umhüllen des Exoskops 1 und zum Ermöglichen eines Luftstroms in den Luftkanälen 16 und ggf. innerhalb des Innenraums 21 sowie zur Handhabung bei der chirurgischen Operation optimiert ist. Die in den Luftkanal 16 eingeblasene Luft strömt innerhalb des Luftkanals 16 in Pfeilrichtung durch den proximalen Abschnitt 13, den Schaftabschnitt 12, den distalen Abschnitt 11, wieder durch den Schaftabschnitt 12 und zurück zum proximalen Abschnitt 13 der Sterilhülle 10, wo sie zur Auslassöffnung gelangt, in der das Filter 22 angeordnet ist. Die vom Exoskop 1 abgegebene Verlustwärme erwärmt die im Innenraum 21 der Sterilhülle 10 befindliche Luft. Die Wärme wird, wie in Fig. 1 durch die gekrümmten Pfeile angedeutet, im Innenraum 21 durch thermische Konvektion zur Innenwand 14 der Sterilhülle 10 transportiert, wo sie von der vom Ventilator 20 geförderten, im Luftkanal 16 strömenden Luft mitgenommen wird; wird vom Ventilator 20 auch Luft in den Innenraum 21 eingeblasen, so wird zumindest ein Teil der Wärme von dieser aufgenommen. Nach Aufnahme der vom Exoskop 1 abgegebenen Wärme tritt die vom Ventilator 20 zugeführte Luft durch das Filter 22 aus dem Luftkanal 16 bzw. dem Innenraum 21 in den Außenraum aus und führt hierdurch die Wärme ab.

Nach Beendigung der Operation werden das Exoskop 1 sowie ggf. der Ventilator 20 für eine erneute Verwendung aus der Sterilhülle 10 entnommen und die Sterilhülle 10 gemeinsam mit dem Filter 22 entsorgt.

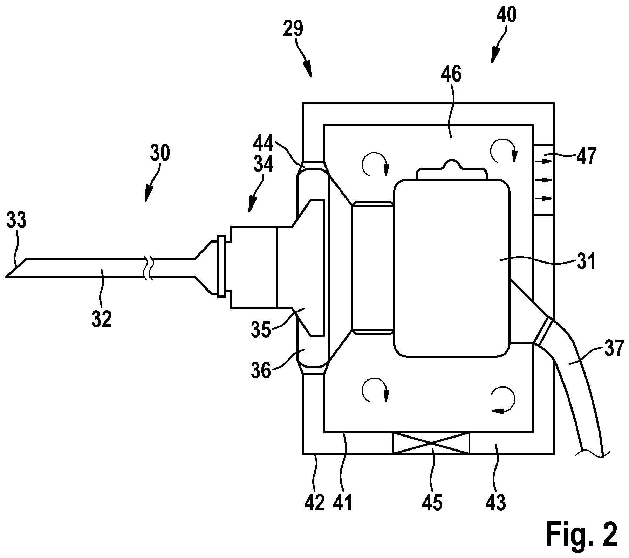

In Fig. 2 ist ein Endoskop 29 gezeigt, das eine Endoskopoptik 30 umfasst, an die ein Kamerakopf 31 angeschlossen ist. Die Endoskopoptik 30 umfasst einen langerstreckten Schaft 32, an dessen distalem Ende 33 ein Endoskopobjektiv angeordnet ist. Das von dem Endoskopobjektiv erzeugte endoskopische Bild wird durch einen in dem Schaft 32 angeordneten Bildweiterleiter, beispielsweise ein geordnetes Faserbündel oder ein oder mehrere Relaislinsensysteme, zu einem im proximalen Endbereich 34 der Endoskopoptik 30 aufgenommenen Okular weitergeleitet. Mit Hilfe der Okularmuschel 35 kann das endoskopische Bild, das von dem Endoskopobjektiv erzeugt und vom Bildweiterleiter übertragen worden ist, betrachtet werden. Zum Betrieb der Endoskopoptik 30 als Videoendoskop ist an die Okularmuschel 35 mit einem Adapter 36 der Kamerakopf 31 angeschlossen, der eine elektronische Kamera zur Aufnahme des endoskopischen Bilds enthält. Ein Verbindungskabel 37 stellt die Verbindung mit einer nicht dargestellten Versorgungs- und Auswerteeinrichtung her.

Da der Kamerakopf 31 nicht ausreichend sterilisierbar ist, ist dieser bei der Verwendung bei einer endoskopischen Operation von einer Sterilhülle 40 umschlossen. Die Sterilhülle 40 ist mit einer Innenwand 41 und einer Außenwand 42 ausgebildet, zwischen denen, sofern die Sterilhülle 40 flexibel ausgebildet ist, eine Stützkammer 43 gebildet wird. Die Sterilhülle 40 ist mit einer Dichtung 44, die eine entsprechende Öffnung umschließt, gasdicht an dem Adapter 36 angeschlossen. Das Versorgungskabel 37 ist gasdicht durch eine weitere Öffnung der Sterilhülle 40 geführt. Ein Ventilator 45 ist zum Zuführen eines Luftstroms in den Innenraum 46 der Sterilhülle 40 und ggf. zum Erzeugen eines Überdrucks in der Stützkammer 43 ausgebildet. Die in den Innenraum 46 eingeblasene Luft tritt durch eine Austrittsöffnung, in die ein Filter 47 eingesetzt ist, wieder aus.

Zur Vorbereitung der Verwendung des Kamerakopfs 31 mit der Endoskopoptik 30 bei einer endoskopischen Operation wird der Kamerakopf 31 mit einer zunächst noch biegeschlaffen Sterilhülle 40 überzogen bzw. in zwei Hälften einer steifen Sterilhülle 40, die luftdicht zusammengesteckt werden können, eingesetzt. Die Sterilhülle 40 wird sodann mit der Dichtung 44 gasdicht an den Adapter 36 angeschlossen, das Verbindungskabel 37 an den Kamerakopf 31 angeschlossen und die Öffnung für das Verbindungskabel 37, die zum Einführen des Kamerakopfs 31 in die Sterilhülle 40 gedient hatte, gasdicht am Verbindungskabel 37 abgeschlossen; ggf. werden der Ventilator 45 und das Filter 47 in die Sterilhülle 40 eingesetzt. Zuvor oder danach wird der Kamerakopf 31 mit dem Adapter 36 auf die Okularmuschel 35 der Endoskopoptik 30 aufgesetzt.

Wird der Kamerakopf 31 in Betrieb genommen, so erzeugt dieser Wärme, die zur Vermeidung einer Überhitzung abgeführt werden muss. Hierfür wird der Ventilator 45 in Betrieb genommen. Der Ventilator 45 fördert Luft in den von der Innenwand 41 umschlossenen Innenraum 46 der Sterilhülle 40, und ggf. in zwischen der Innenwand 41 und der Außenwand 42 gebildete Stützkammern 43. Sofern die Sterilhülle 40 flexibel ist, stabilisiert der in der Stützkammer 43 erzeugte Überdruck die Sterilhülle 40 in einer vorgegebenen Form, die zum Umhüllen des Kamerakopfs 31, zum Ermöglichen einer Luftströmung im Innenraum 46 und zur Handhabung des Endoskops 29 optimiert ist. Die in den Innenraum 46 eingeblasene Luft nimmt die vom Kamerakopf 31 abgegebene Wärme auf, strömt zur Auslassöffnung, in der das Filter 47 angeordnet ist und tritt durch dieses aus der Sterilhülle 40 aus. Wie durch die gekrümmten Pfeile angedeutet ist, strömt die Luft turbulent, so dass ein intensiverer Wärmeübergang an den wärmeabgebenden Oberflächen des Kamerakopfs 31 gewährleistet ist. Hierdurch wird die vom Kamerakopf 31 abgegebene Wärme in den Außenraum transportiert.

Nach Beendigung der Operation wird der Kamerakopf 31 von der Endoskopoptik getrennt, der Kamerakopf 31 sowie ggf. der Ventilator 45 aus der Sterilhülle 40 entnommen und die Sterilhülle 40 gemeinsam mit dem Filter 47 entsorgt.

### Bezugszeichenliste

- 1: Exoskop
- 2: Kopf
- 3: Kameraeinheit
- 4: Schaft
- 5: Versorgungskabel
- 6: Haltearm
- 7: Struktur
- 8: Objektfeld
- 9: Lichtkegel
- 10: Sterilhülle
- 11: Distaler Abschnitt
- 12: Schaftabschnitt
- 13: Proximaler Abschnitt
- 14: Innenwand
- 15: Außenwand
- 16: Luftkanal
- 17: Kunststoffeinsatz
- 18: Deckglas
- 20: Ventilator
- 21: Innenraum
- 22: Filter
- 29: Endoskop
- 30: Endoskopoptik
- 31: Kamerakopf
- 32: Schaft
- 33: Distales Ende
- 34: Proximaler Endbereich
- 35: Okularmuschel
- 36: Adapter
- 37: Verbindungskabel
- 40: Sterilhülle
- 41: Innenwand
- 42: Außenwand
- 43: Stützkammer
- 44: Dichtung
- 45: Ventilator
- 46: Innenraum
- 47: Filter

## Patentansprüche

1. Sterilhülle für ein medizinisches Beobachtungsinstrument, insbesondere für ein Endoskop, Exoskop (1) oder Operationsmikroskop, die zum Umhüllen des medizinischen Beobachtungsinstruments ausgebildet ist, wobei die Sterilhülle (10) einen Lufteinlass, einen Luftauslass und Mittel zum Fördern und/oder Führen eines Luftstroms vom Lufteinlass durch die Sterilhülle (10) zum Luftauslass zur Abführung der von mindestens einer wärmeabgebenden Komponente des medizinischen Beobachtungsinstruments abgegebenen Wärme aufweist und wobei der Luftaustritt ein Filter (22) aufweist, **dadurch gekennzeichnet, dass** die Sterilhülle (10) ein an einem distalen Fenster des Beobachtungsinstruments fixierbares Deckglas (18) aufweist.

2. Sterilhülle nach Anspruch 1, wobei die Mittel zum Fördern und/oder Führen des Luftstroms mindestens einen Ventilator (20) und/oder mindestens eine Pumpe umfassen.

3. Sterilhülle nach Anspruch 2, wobei die Sterilhülle (10) eine elektrische Energiequelle zur Versorgung eines Antriebs des Ventilators (20) bzw. der Pumpe umfasst.

4. Sterilhülle nach Anspruch 2 oder 3, wobei der Ventilator (20) bzw. die Pumpe und/oder die elektrische Energiequelle lösbar mit der Sterilhülle (10) verbunden ist bzw. sind.

5. Sterilhülle nach einem der vorhergehenden Ansprüche, wobei die Sterilhülle (10) eine Mehrzahl von miteinander und mit dem Lufteinlass und dem Luftauslass kommunizierenden Luftführungskammern aufweist.

6. Sterilhülle nach einem der vorhergehenden Ansprüche, wobei die Sterilhülle (10) zumindest bereichsweise eine Innenwand (14) und eine Außenwand (15) aufweist, wobei im Zwischenraum zwischen der Innenwand (14) und der Außenwand (15) mindestens eine Luftführungskammer zum Führen zumindest eines Teils des Luftstroms ausgebildet ist.

7. Sterilhülle nach Anspruch 5 oder 6, wobei der Luftstrom in der mindestens einen Luftführungskammer turbulent geführt ist.

8. Sterilhülle nach einem der vorhergehenden Ansprüche, wobei die Sterilhülle zumindest bereichsweise flexibel ausgebildet ist und in der Sterilhülle (10) ein für eine Stabilisierung der Sterilhülle (10) geeigneter Überdruck erzeugbar ist.

9. Sterilhülle nach Anspruch 8, wobei die Sterilhülle (10) zumindest bereichsweise eine Innenwand (14) und eine Außenwand (15) aufweist, wobei im Zwischenraum zwischen der Innenwand (14) und der Außenwand (15) mindestens eine unter einen Überdruck setzbare Stützkammer zum Stabilisieren der Sterilhülle (10) ausgebildet ist.

10. Sterilhülle nach einem der vorhergehenden Ansprüche, wobei die Sterilhülle (10) eine selbststabilisierende Stützstruktur aufweist und/oder zumindest bereichsweise steif ausgebildet ist.

11. Sterilhülle nach einem der vorhergehenden Ansprüche, wobei der Luftaustritt ein Ventil aufweist.

12. Sterilhülle nach einem der vorhergehenden Ansprüche, wobei der Luftaustritt von einem mit dem endoskopischen Beobachtungsinstrument beobachtbaren Objektfeld (8) entfernt und/oder fortweisend angeordnet ist.

13. Sterilhülle nach einem der vorhergehenden Ansprüche, wobei die Sterilhülle (10) zumindest teilweise aus einem luftdurchlässigen Material besteht.

14. Verfahren zum Betreiben eines medizinischen Beobachtungsinstruments, insbesondere eines Exoskops (1) oder Operationsmikroskops, wobei das medizinische Beobachtungsinstrument von einer Sterilhülle (10) nach einem der vorhergehenden Ansprüche umhüllt wird, das medizinische Beobachtungsinstrument zur Beobachtung eines Operationssitus in geeignetem Abstand hierüber angeordnet wird, eine wärmeabgebende Komponente des medizinischen Beobachtungsinstruments betrieben wird und ein Luftstrom vom Lufteinlass durch die Sterilhülle (10) zum Luftauslass zur Abführung der von der wärmeabgebenden Komponente abgegebenen Wärme gefördert und/oder geführt wird, wobei der Luftaustritt ein Filter (22) aufweist.

## Claims

1. Sterile sheath for a medical observation instrument, in particular for an endoscope, an exoscope (1), or a surgical microscope, which is designed to envelop the medical observation instrument, wherein the sterile sheath (10) comprises an air inlet, an air outlet and means for conveying and/or guiding an airflow from the air inlet, through the sterile sheath (10), to the air outlet in order to dissipate the heat emitted by the at least one heat-emitting component of the medical observation instrument, and wherein the air outlet comprises a filter (22), **characterized in that** the sterile sheath (10) comprises a glass cover (18) fixable on the distal window of the observation instrument.

2. Sterile sheath according to claim 1, wherein the means for conveying and/or guiding the airflow comprise at least one fan (20) and/or at least one pump.

3. Sterile sheath according to claim 2, wherein the sterile sheath (10) comprises an electrical energy source for supplying a drive of the fan (20) or the pump.

4. Sterile sheath according to claim 2 or 3, wherein the fan (20) or the pump and/or the electrical energy source is or are detachably connected to the sterile sheath (10).

5. Sterile sheath according to one of the preceding claims, wherein the sterile sheath (10) comprises a plurality of air guidance chambers communicating with one another and with the air inlet and the air outlet.

6. Sterile sheath according to one of the preceding claims, wherein, at least in some sections, the sterile sheath (10) comprises an inner wall (14) and an outer wall (15), wherein at least one air guidance chamber for guiding at least a portion of the airflow is formed in the intermediate space between the inner wall (14) and the outer wall (15).

7. Sterile sheath according to claim 5 or 6, wherein the airflow in the at least one air guidance chamber is guided in a turbulent manner.

8. Sterile sheath according to one of the preceding claims, wherein, at least in some sections, the sterile sheath is designed to be flexible, and a positive pressure suitable for a stabilization of the sterile sheath (10) can be generated in the sterile sheath (10).

9. Sterile sheath according to claim 8, wherein, at least in some sections, the sterile sheath (10) comprises an inner wall (14) and an outer wall (15), wherein at least one support chamber that is pressurizable to a positive pressure is formed in the intermediate space between the inner wall (14) and the outer wall (15) in order to stabilize the sterile sheath (10).

10. Sterile sheath according to one of the preceding claims, wherein the sterile sheath (10) comprises a self-stabilizing supporting structure and/or is, at least in some sections, designed to be rigid.

11. Sterile sheath according to one of the preceding claims, wherein the air outlet comprises a valve.

12. Sterile sheath according to one of the preceding claims, wherein the air outlet is arranged at a distance from and/or facing away from an object field (8) observable by the endoscopic observation instrument.

13. Sterile sheath according to one of the preceding claims, wherein, at least in some sections, the sterile sheath (10) consists of an air-permeable material.

14. Method of operating a medical observation instrument, in particular an exoscope (1) or a surgical microscope, wherein the medical observation instrument is enveloped by a sterile sheath (10) according to one of the preceding claims, the medical observation instrument for observing a surgical site is arranged at a suitable distance therefrom, a heat-emitting component of the medical observation instrument is operated, and an airflow is conveyed and/or guided from the air inlet, through the sterile sheath (10), to the air outlet in order to dissipate the heat emitted by the heat-emitting component, wherein the air outlet comprises a filter (22).

## Revendications

1. Enveloppe stérile pour un instrument d'observation médicale, en particulier pour un endoscope, exoscope (1) ou microscope chirurgical, qui est conçue pour envelopper l'instrument d'observation médicale, l'enveloppe stérile (10) comportant une entrée d'air, une sortie d'air et des moyens pour transporter et/ou guider un flux d'air depuis l'entrée d'air, à travers l'enveloppe stérile (10), jusqu'à la sortie d'air pour dissiper la chaleur émise par au moins un composant émettant de la chaleur de l'instrument d'observation médicale et la sortie d'air comportant un filtre (22), **caractérisée en ce que** l'enveloppe stérile (10) comporte une lamelle couvre-objet (18) pouvant être fixée sur une fenêtre distale de l'instrument d'observation.

2. Enveloppe stérile selon la revendication 1, les moyens pour transporter et/ou guider le flux d'air comprenant au moins un ventilateur (20) et/ou au moins une pompe.

3. Enveloppe stérile selon la revendication 2, l'enveloppe stérile (10) comprenant une source d'énergie électrique pour alimenter un entraînement du ventilateur (20) ou de la pompe.

4. Enveloppe stérile selon la revendication 2 ou 3, le ventilateur (20) ou la pompe et/ou la source d'énergie électrique étant relié(e)(s) de manière détachable à l'enveloppe stérile (10).

5. Enveloppe stérile selon l'une des revendications précédentes, l'enveloppe stérile (10) comportant une pluralité de chambres de guidage d'air communiquant les unes avec les autres et avec l'entrée d'air et la sortie d'air.

6. Enveloppe stérile selon l'une des revendications précédentes, l'enveloppe stérile (10) comportant, au moins dans certaines zones, une paroi intérieure (14) et une paroi extérieure (15), au moins une chambre de guidage d'air pour guider au moins une partie du flux d'air étant réalisée dans l'espace intermédiaire entre la paroi intérieure (14) et la paroi extérieure (15).

7. Enveloppe stérile selon la revendication 5 ou 6, le flux d'air étant guidé de manière turbulente dans l'au moins une chambre de guidage d'air.

8. Enveloppe stérile selon l'une des revendications précédentes, l'enveloppe stérile étant réalisée flexible au moins dans certaines zones et une surpression adaptée pour une stabilisation de l'enveloppe stérile (10) pouvant être générée dans l'enveloppe stérile (10).

9. Enveloppe stérile selon la revendication 8, l'enveloppe stérile (10) comportant, au moins dans certaines zones, une paroi intérieure (14) et une paroi extérieure (15), au moins une chambre de support susceptible d'être mise en surpression étant réalisée dans l'espace intermédiaire entre la paroi intérieure (14) et la paroi extérieure (15) pour stabiliser l'enveloppe stérile (10).

10. Enveloppe stérile selon l'une des revendications précédentes, l'enveloppe stérile (10) comportant une structure de support autostabilisante et/ou étant réalisée rigide au moins dans certaines zones.

11. Enveloppe stérile selon l'une des revendications précédentes, la sortie d'air comportant une valve.

12. Enveloppe stérile selon l'une des revendications précédentes, la sortie d'air étant disposée à distance et/ou orientée à l'opposé d'un champ d'objet (8) observable avec l'instrument d'observation endoscopique.

13. Enveloppe stérile selon l'une des revendications précédentes, l'enveloppe stérile (10) étant constituée au moins en partie d'un matériau perméable à l'air.

14. Procédé de fonctionnement d'un instrument d'observation médicale, en particulier d'un exoscope (1) ou microscope chirurgical, l'instrument d'observation médicale étant enveloppé par une enveloppe stérile (10) selon l'une des revendications précédentes, l'instrument d'observation médicale étant disposé pour l'observation d'un site opératoire à une distance adaptée au-dessus de celui-ci, un composant émettant de la chaleur de l'instrument d'observation médicale étant exploité et un flux d'air étant transporté et/ou guidé depuis l'entrée d'air, à travers l'enveloppe stérile (10), jusqu'à la sortie d'air pour dissiper la chaleur émise par le composant émettant de la chaleur, la sortie d'air comportant un filtre (22).
